# EUROPEAN PATENT APPLICATION

(11) **EP 4 180 516 A1**
(43) Date of publication of application: **17.05.2023**
(21) Application number: 21842746.6
(22) Date of filing: 13.07.2021
(51) Int. Cl.: C12N 5/077, A61K 35/34, A61P 21/04, C12N 5/10, C12N 15/12

(54) **SKELETAL MUSCLE PRECURSOR CELLS AND METHOD FOR PURIFYING SAME, COMPOSITION FOR TREATING MYOGENIC DISEASES, AND METHOD FOR PRODUCING CELL GROUP CONTAINING SKELETAL MUSCLE PRECURSOR CELLS**

(30) Priority: 13.07.2020 JP 2020120226
(71) Applicant: Kyoto University, Kyoto-shi, Kyoto 606-8501 (JP); Asahi Kasei Kabushiki Kaisha, Tokyo 1000006 (JP)
(72) Inventor: SAKURAI, Hidetoshi, Kyoto-shi, Kyoto 606-8501 (JP); ZHAO, Mingming, Kyoto-shi, Kyoto 606-8501 (JP); KATO, Hiroki, Tokyo 100-0006 (JP); TAZUMI, Atsutoshi, Tokyo 100-0006 (JP)
(74) Representative: dompatent von Kreisler Selting Werner - Partnerschaft von Patent- und Rechtsanwälten mbB
(86) International application number: PCT/JP2021/026344
(87) International publication number: WO 2022/014604

(57) **Abstract**

The present invention provides a composition that is for treating myogenic diseases and that contains a cell group including skeletal muscle precursor cells differentiation-induced from pluripotent stem cells, the composition being characterized in that the cell group includes the skeletal muscle precursor cells that are Pax7-positive, and are CD146 (MCAM)-positive and CD57-negative on the surface thereof. The present invention also provides a method for purifying skeletal muscle precursor cells.

## Description

### FIELD

The present invention relates to skeletal muscle progenitor cells, and to a composition for treating myogenic disease comprising the same. The invention further relates to a method for purifying skeletal muscle progenitor cells and a method for producing a cell group comprising skeletal muscle progenitor cells. The present application claims priority based on Japanese Patent Application No. 2020-120226 filed on July 13, 2020, the entirety of which is incorporated herein by reference.

### BACKGROUND

Since the first reports of embryonic stem cells (ES cells) and cells with pluripotency such as induced pluripotent stem cells (iPS cells) obtained by introducing specific genes of undifferentiated cells into somatic cells, there has been increased focus on treatment methods involving transplantation of skeletal muscle progenitor cells that have been induced to differentiate from pluripotent stem cells, as treatment methods for myogenic disease, and especially treatment methods for muscular dystrophy.

Previously known methods for inducing differentiation of skeletal muscle progenitor cells from pluripotent stem cells include (1) a method of growing a single human ES cell by suspension culture and culturing the proliferated cells in serum-free culture medium by adhesion culture, followed by isolation and further culturing of the CD73-positive cells, and isolation and proliferation of the NCAM-positive cells (NPL 1), (2) a method of culturing human ES cells treated with 5-azacytidine (a demethylating agent) by suspension culture to form an embryo body, followed by further adhesion culture (NPL 2), (3) a method of culturing pluripotent stem cells by suspension culture followed by adhesion culture, and subsequent dissociation with further adhesion culture (PTL 1), and (4) a method of culturing pluripotent stem cells in culture medium containing TGF-β inhibitor, GSK3β inhibitor, HGF, bFGF and IGF1 (PTL 2). Research on clinical applications of skeletal muscle progenitor cells induced by these methods is currently ongoing.

When skeletal muscle progenitor cells are induced to differentiate from pluripotent stem cells, the differentiated cell population also includes undifferentiated pluripotent stem cells and cells other than skeletal muscle progenitor cells, and consequently their use in the clinic requires purifying methods to increase the yield and purity of the desired skeletal muscle progenitor cells. A known method exists for purifying skeletal muscle progenitor cells that have been induced to differentiate from pluripotent stem cells, wherein the cell surface markers CD82 and CD57 are used to isolate CD82-positive and CD57-negative cells (NPL 3).

### [CITATION LIST]

### [PATENT LITERATURE]

[PTL 1] Japanese Patent Public Inspection No. 2013-527746
[PTL 2] International Patent Publication No. WO2016/108288

### [NON PATENT LITERATURE]

[NPL 1] Barberi T, et al. Nat Med. 13:642-8, 2007
[NPL 2] Zheng JK, et al. Cell Res. 16:713-22, 2006
[NPL 3] Matthew S. Alexander, et al., Cell Stem Cell 19, 1, 2016

### SUMMARY

### [TECHNICAL PROBLEM]

It is an object of the invention to provide skeletal muscle progenitor cells of quality suited for clinical applications, as well as a method of purifying the cells.

### [SOLUTION TO PROBLEM]

After much diligent research directed toward achieving the object stated above, the present inventors have succeeded in increasing the yield and purity of skeletal muscle progenitor cells of quality suited for clinical applications, by a purifying method using a specific cell surface marker. The present inventors have further found that skeletal muscle progenitor cells obtained by the method, or a composition containing them, exhibit a highly notable effect of regenerating damaged muscular fibers without inducing fibrosis at the site of their application in a living body, and the invention has been completed.

Specifically, the present invention provides the following.
[1] Skeletal muscle progenitor cells that are positive for Pax7, and that are positive for CD146 (MCAM) and negative for CD57 on the cell surfaces.
[2] The skeletal muscle progenitor cells of [1] above, which are derived from pluripotent stem cells.
[3] The skeletal muscle progenitor cells of [2] above, wherein the pluripotent stem cells are selected from the group consisting of ES cells, ntES cells and iPS cells.
[4] The skeletal muscle progenitor cells of any one of [1] to [3], wherein the skeletal muscle progenitor cells are capable of differentiating into myosin heavy chain (MHC)-positive skeletal muscle cells and further maturing into multinucleated skeletal muscle cells in a culture system.
[5] A composition for treating myogenic disease, which includes a cell group comprising skeletal muscle progenitor cells of any one of [1] to [4].
[6] The composition of [5] above, wherein the cell group comprises 30% or more of the skeletal muscle progenitor cells.
[7] The composition of [5] or [6] above, which does not induce fibrosis at the site of application in a living body.
[8] The composition of any one of [5] to [7] above, wherein the cell group is derived from pluripotent stem cells.
[9] The composition of [8] above, wherein the pluripotent stem cells are selected from the group consisting of ES cells, ntES cells and iPS cells.
[10] The composition of any one of [5] to [9] above, wherein the myogenic disease is muscular dystrophy.
[11] The composition of any one of [5] to [10] above, which includes a pharmaceutically acceptable carrier.
[12] A method for purifying skeletal muscle progenitor cells, the method comprising:
   a step of recovering a cell group that is positive for CD146 (MCAM) and negative for CD57 on the cell surfaces, from a cell population comprising skeletal muscle progenitor cells.
[13] The method of [12] above, wherein the cell group comprises 30% or more of the Pax7-positive skeletal muscle progenitor cells.
[14] The method of [12] or [13] above, wherein the step is performed by a fluorescence-activated cell sorting (FACS) or magnetic cell sorting (MACS) method.
[15] The method of any one of [12] to [14] above, wherein the cell population is derived from pluripotent stem cells.
[16] The method of [15] above, wherein the pluripotent stem cells are selected from the group consisting of ES cells, ntES cells and iPS cells.
[17] A kit for use in the method of any one of [12] to [16] above, the kit comprising a binding agent which specifically binds CD146 (MCAM) and a binding agent which specifically binds CD57.
[18] A method for producing a cell group comprising skeletal muscle progenitor cells, the method comprising:
   a step of recovering a cell group that is positive for CD146 (MCAM) and negative for CD57 on the cell surfaces, from a cell population comprising skeletal muscle progenitor cells.
[19] The method of [18] above, wherein the cell group comprises 30% or more of the Pax7-positive skeletal muscle progenitor cells.
[20] The method of [18] or [19] above, wherein the step is performed by a fluorescence-activated cell sorting (FACS) or magnetic cell sorting (MACS) method.
[21] The method of any one of [18] to [20] above, wherein the cell population is derived from pluripotent stem cells.
[22] The method of [21] above, wherein the pluripotent stem cells are selected from the group consisting of ES cells, ntES cells and iPS cells.
[23] A kit for use in the method of any one of [18] to [22] above, the kit comprising a binding agent which specifically binds CD146 (MCAM) and a binding agent which specifically binds CD57.
[24] A cell group comprising skeletal muscle progenitor cells, obtained by the method of any one of [18] to [22].
[25] A cell group for use in treatment of myogenic disease, the cell group comprising skeletal muscle progenitor cells that are Pax7-positive and are positive for CD146 (MCAM) and negative for CD57 on the cell surfaces.
[26] The use of a cell group for production of a drug for treatment of myogenic disease,
   wherein the cell group comprises skeletal muscle progenitor cells that are Pax7-positive and are positive for CD146 (MCAM) and negative for CD57 on the cell surfaces.
[27] A method for treatment of a subject suffering from myogenic disease, the method comprising:
   administration of a cell group comprising skeletal muscle progenitor cells that are Pax7-positive and are positive for CD146 (MCAM) and negative for CD57 on the cell surfaces, to a subject in need thereof.

### [ADVANTAGEOUS EFFECTS OF INVENTION]

The skeletal muscle progenitor cells provided by the invention can treat a subject suffering from myogenic disease without inducing fibrosis at the site of application in a living body.

### BRIEF DESCRIPTION OF DRAWINGS

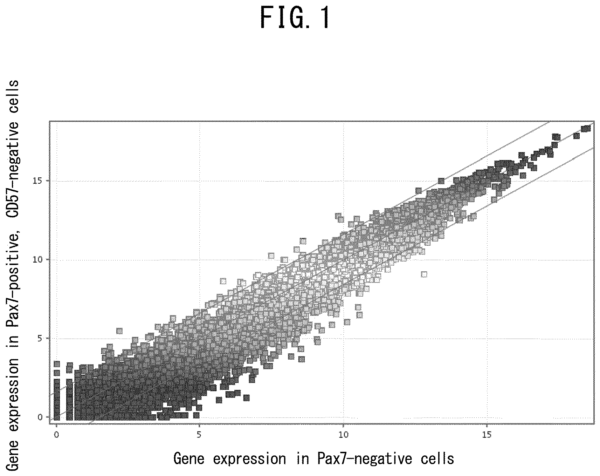

Fig. 1 shows results of measuring gene expression levels with RNA-seq, after selecting Pax7-positive, CD57-negative cells and Pax7-negative cells by FACS analysis from among a cell population comprising skeletal muscle progenitor cells obtained by inducing differentiation of human iPS cells (s01-Pax7-Venus) expressing the fluorescent protein Venus in linkage with Pax7 expression.

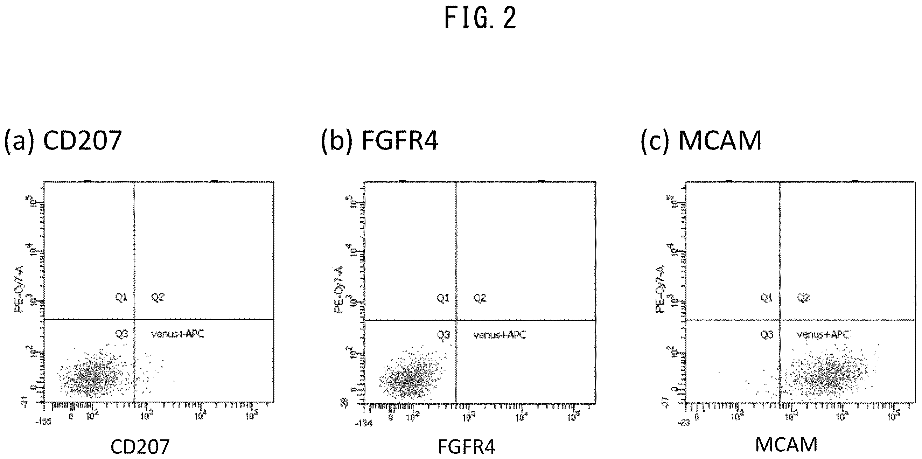

Fig. 2 shows results of analyzing expression of cell surface proteins in Pax7-positive cells selected by FACS analysis from among a cell population comprising skeletal muscle progenitor cells obtained by inducing differentiation of human iPS cells (s01-Pax7-Venus) expressing the fluorescent protein Venus in linkage with Pax7 expression. Analysis was with antibodies for CD207 (a), FGFR4 (b) and MCAM (c).

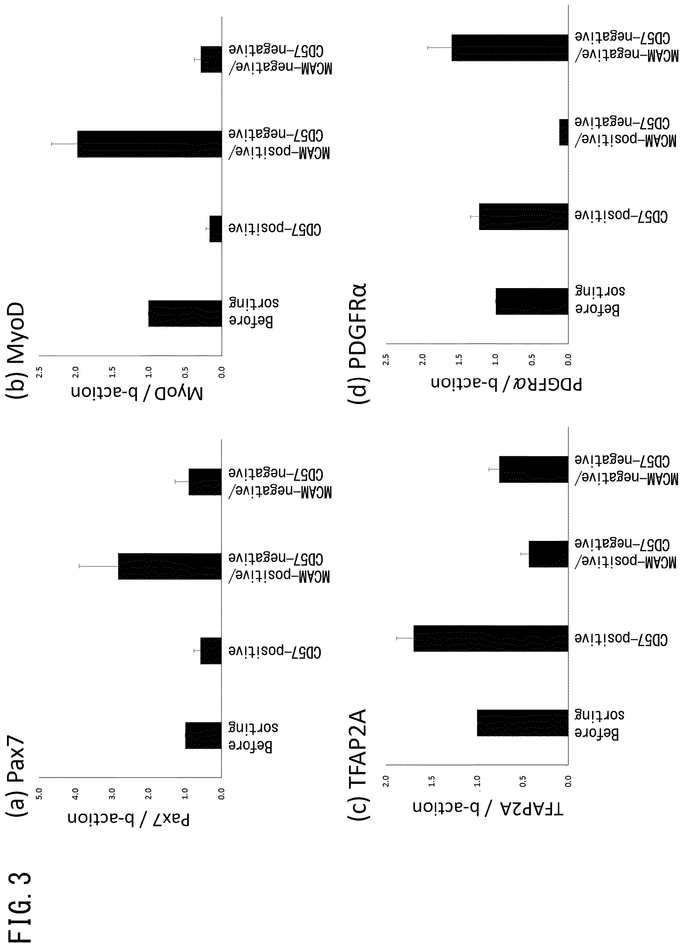

Fig. 3 shows results of quantifying gene expression levels after selecting CD57-positive cells, MCAM-positive, CD57-negative cells and MCAM-negative, CD57-negative cells by FACS from among a cell population comprising skeletal muscle progenitor cells obtained by inducing differentiation of human iPS cells. The results are for the Pax7 gene (a), the MyoD gene (b), the TFAP2A gene (c) and the PDGFRα gene (d). The data are percentages with respect to the expression level in bulk (before selection), as mean ±SE.

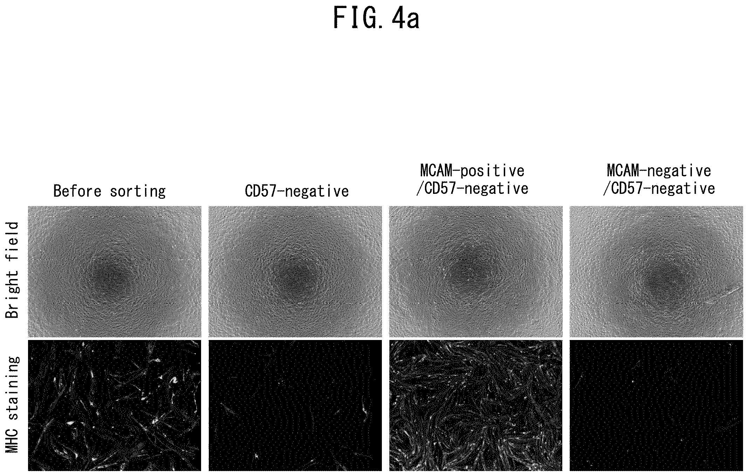

Fig. 4a shows results of selecting CD57-positive cells, MCAM-positive, CD57-negative cells and MCAM-negative, CD57-negative cells by FACS from among a cell population comprising skeletal muscle progenitor cells obtained by inducing differentiation of human iPS cells, and observing and quantifying expression of the Myosin Heavy Chain (MHC) after inducing their differentiation to myotube cells. The results for the bright field and for immunostaining with MHC antibody are shown.

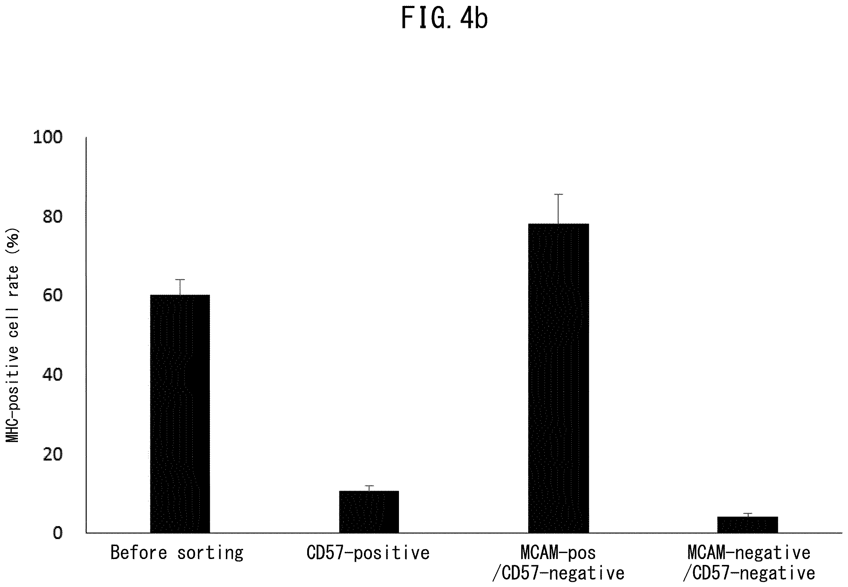

Fig. 4b shows results of selecting CD57-positive cells, MCAM-positive, CD57-negative cells and MCAM-negative, CD57-negative cells by FACS from among a cell population comprising skeletal muscle progenitor cells obtained by inducing differentiation of human iPS cells, and observing and quantifying expression of the Myosin Heavy Chain (MHC) after inducing their differentiation to myotube cells. The results of quantifying the MHC-positive cell percentages calculated from Fig. 4a, are shown. The data are represented as mean ±SE.

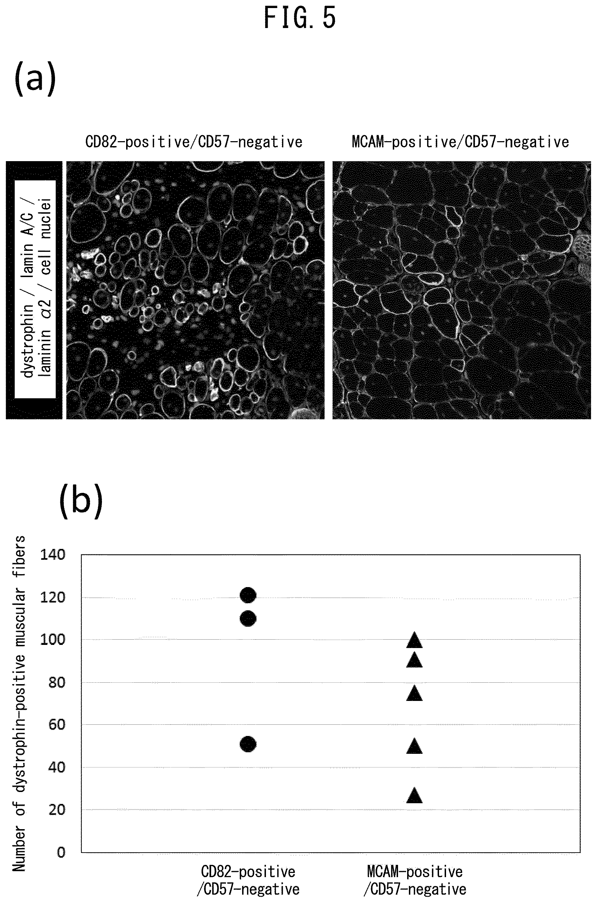

Fig. 5 shows results of tissue imaging four weeks after selecting CD82-positive, CD57-negative cells and MCAM-positive, CD57-negative cells by FACS from among a cell population comprising skeletal muscle progenitor cells obtained by inducing differentiation of human iPS cells, and transplanting them into the gastrocnemius muscle of muscular dystrophy (DMD/NSG) mice with severe immunodeficiency. Immunostained images for dystrophin (green), laminin α2 (white), lamin A/C (red) and cell nuclei (blue) at the transplanted site, are shown. The number of dystrophin-positive muscular fibers is shown.

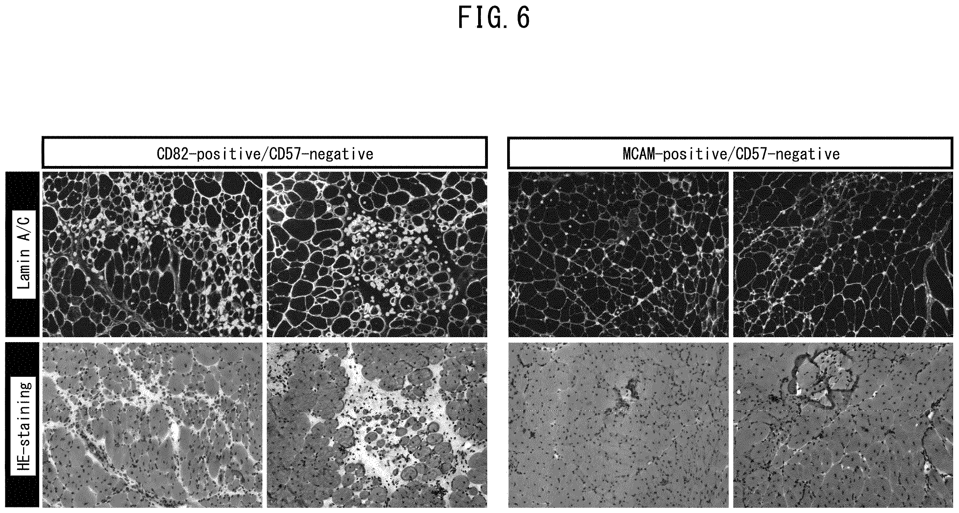

Fig. 6 shows results of tissue imaging four weeks after selecting CD82-positive, CD57-negative cells or MCAM-positive, CD57-negative cells by FACS from among a cell population comprising skeletal muscle progenitor cells obtained by inducing differentiation of human iPS cells, and transplanting them into the gastrocnemius muscle of muscular dystrophy (DMD/NSG) mice with severe immunodeficiency. The upper row shows immunostained images for lamin A/C (green) and laminin α2 (white), and the lower row shows HE stained images.

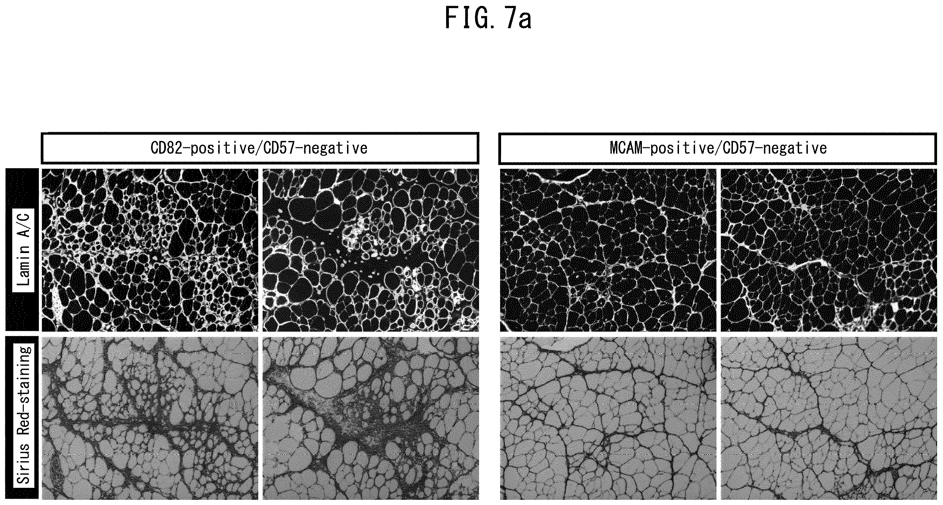

Fig. 7a shows results of tissue imaging four weeks after selecting CD82-positive, CD57-negative cells or MCAM-positive, CD57-negative cells by FACS from among a cell population comprising skeletal muscle progenitor cells obtained by inducing differentiation of human iPS cells, and transplanting them into the gastrocnemius muscle of muscular dystrophy (DMD/NSG) mice with severe immunodeficiency. The upper row shows immunostained images for lamin A/C (green) and laminin α2 (white), and the lower row shows Sirius Red stained images.

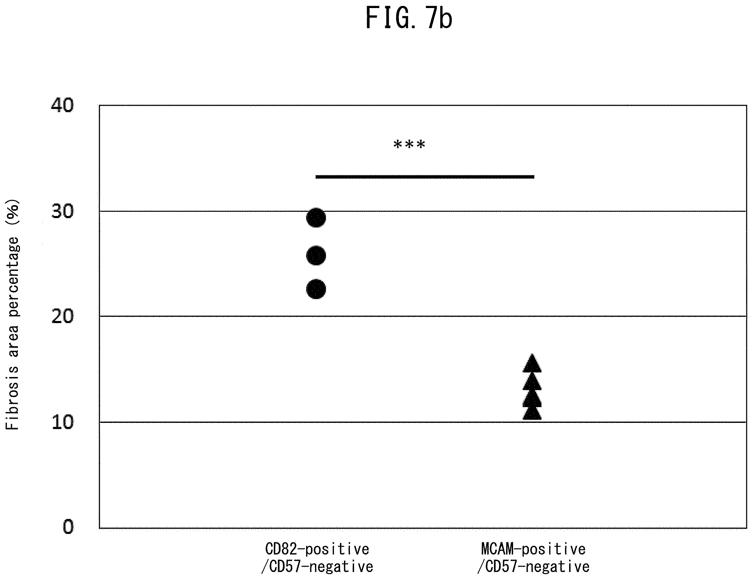

Fig. 7b shows results of tissue imaging four weeks after selecting CD82-positive, CD57-negative cells or MCAM-positive, CD57-negative cells by FACS from among a cell population comprising skeletal muscle progenitor cells obtained by inducing differentiation of human iPS cells, and transplanting them into the gastrocnemius muscle of muscular dystrophy (DMD/NSG) mice with severe immunodeficiency. The percentage of fibrosis area with respect to tissue area, as calculated from Fig. 7(a), is shown. Statistical analysis was carried out by t test. ∗∗∗:P <0.005

### DESCRIPTION OF EMBODIMENTS

The present invention will now be explained in greater detail.

Unless otherwise defined, the terms (technical and scientific terms) used herein have the same meanings as generally understood by those skilled in the art.

The present invention relates to skeletal muscle progenitor cells and a composition for treating myogenic disease comprising the same, as well as a method for purifying skeletal muscle progenitor cells, a method for producing a cell group comprising skeletal muscle progenitor cells and a method for treating a subject suffering from myogenic disease.

As used herein, the term "skeletal muscle" refers to mature muscle, and includes muscle fibers, i.e. multinucleated muscle cells. Also as used herein, the term "skeletal muscle progenitor cells" used in a context referring to ordinary skeletal muscle progenitor cells, means cells that have not yet fully reached the stage of matured muscle cells but are at an earlier stage and still have the ability to selectively differentiate to muscle cells, and does not mean that they have absolutely no ability to differentiate into other mesodermal cells such as osteoblasts and adipocytes, and therefore "skeletal muscle progenitor cells" may also include cells having the ability to differentiate into cells other than muscle cells. Skeletal muscle progenitor cells are generally characterized by expression of specific genes, and they can be identified by detecting expression of marker genes such as MyoD, Myf5, Pax7, Myogenin, myosin heavy chain (MHC), NCAM, Desmin, SkMAct, M-Cadherin, Fgfr4 and VCAME1, for example. The skeletal muscle progenitor cells to be used for the invention are characterized at least by being Pax7-positive, and being positive for CD146 (MCAM) and negative for CD57 on the cell surfaces, and when applied to a living body they exhibit a very excellent effect without inducing fibrosis at the site to which they are applied. The skeletal muscle progenitor cells of the invention are capable of differentiating into myosin heavy chain (MHC)-positive skeletal muscle cells and further maturing into multinucleated skeletal muscle cells in a culture system. When the skeletal muscle progenitor cells of the invention are transplanted, therefore, they efficiently mature into skeletal muscle cells in the transplanted tissue site to exhibit a therapeutic effect.

Unless otherwise specified, the skeletal muscle progenitor cells of the invention include skeletal muscle stem cells and/or satellite cells.

The term "skeletal muscle stem cells", as used herein, refers to cells that have self-renewal ability, differentiate to myoblasts and proliferate when skeletal muscle undergoes damage, and fuse with damaged muscular fibers to carry out repair, and "satellite cells" that are observed in matured muscle are included within the term "skeletal muscle stem cells". Skeletal muscle stem cells and satellite cells are known to express Pax7 as an expression marker.

According to the invention, "CD146" which is used as a surface marker is a cell adhesion molecule that is known as melanoma cell adhesion molecule (MCAM) or cell surface glycoprotein MUC18 (these are referred to herein as "CD146", "MCAM" or "CD146 (MCAM)" and are synonymous). In humans, CD146 is encoded by the MCAM gene, and the human CD146 mRNA and amino acid sequences are available in the GenBank database and GenPept database as Accession No. NM_006500 and NP_006491, with no particular limitation to these, however.

As used herein, the phrase "cells that are positive for CD146 (MCAM) on the cell surfaces" (or "CD146-positive cells") refers to cells that react with a CD146-specific binding agent (such as anti-CD146 antibody) on the cell surfaces, and they can be identified by a publicly known immunological method (such as detection by flow cytometry using anti-CD146 antibody). CD146-positive cells therefore include not only cells expressing wild type CD146 on the cell surfaces, but also cells that express mutant CD146 on the cell surfaces that still react with anti-CD146 antibody.

For the purpose of the invention, "CD57" used as a surface marker is an enzyme known as galactosylgalactosylxylosylprotein 3-beta-glucuronosyltransferase 1 (B3GAT1) or LEU7, and it is a cell surface protein encoded by the B3GAT1 gene. The mRNA and amino acid sequences for human CD57 are available in the GenBank database and GenPept database as Accession No. NM_018644, NM_054025 or NM_001367973, and NP_061114, NP_473366 and NP_001354902, for example, with no particular limitation to these, however.

As used herein, the phrase "cells that are negative for CD57 on the cell surfaces" (or "CD57-negative cells") refers to cells that do not react with a CD57-specific binding agent (such as anti-CD57 antibody) on the cell surfaces, and they can be identified by a publicly known immunological method (such as detection by flow cytometry using anti-CD57 antibody). CD57-negative cells therefore include not only cells that do not express wild type CD57 on the cell surfaces, but also cells that do not express mutant CD57 on the cell surfaces that react with anti-CD57 antibody.

As used herein, "Pax7" is a transcription factor known as a marker for skeletal muscle progenitor cells and especially skeletal muscle stem cells (satellite cells). The mRNA and amino acid sequences for human Pax7 are available in the GenBank database and GenPept database as Accession No. NM_013945, NM_001135254 or NM_002584, and NP_001128726, NP_002575 and NP_039236, for example, with no particular limitation to these, however. Expression of "Pax7" in the skeletal muscle progenitor cells of the invention can be quantified using a publicly known gene expression detecting method.

Conventionally known methods for purifying skeletal muscle progenitor cells from cell populations comprising skeletal muscle progenitor cells that have been induced to differentiate from pluripotent stem cells, include methods for selecting CD82-positive, CD57-negative cells which express CD82 on the cell surfaces and do not express CD57 on the cell surfaces. However, because skeletal muscle progenitor cells purified by such methods produce insufficient yields and purity for Pax7-positive cells and are also contaminated by large amounts of cells other than skeletal muscle progenitor cells, they have been associated with problems such as fibrosis at transplanted tissue sites in most cases. However, the skeletal muscle progenitor cells provided by the invention, and a cell group containing them, have the superior feature of not inducing fibrosis at the site of application in a living body.

According to one embodiment, the invention provides a composition for treating myogenic disease that comprises a cell group comprising skeletal muscle progenitor cells that are Pax7-positive and are positive for CD146 (MCAM) and negative for CD57 on the cell surfaces.

The cell group comprising skeletal muscle progenitor cells in the composition of the invention may also be a cell group comprising other types of cells together with the skeletal muscle progenitor cells. For this embodiment, the percentage of skeletal muscle progenitor cells with respect to the cell group of the invention is preferably 30% or higher, and for example, it may be 30% or higher, 35% or higher, 40% or higher, 45% or higher, 50% or higher, 55% or higher, 60% or higher, 65% or higher, 70% or higher, 75% or higher, 80% or higher, 85% or higher, 90% or higher, 95% or higher, or 100%.

According to one embodiment, the skeletal muscle progenitor cells in the composition of the invention are capable of differentiating into myosin heavy chain (MHC)-positive skeletal muscle cells and further maturing into multinucleated skeletal muscle cells in a culture system. When the composition of the invention has been transplanted, therefore, they efficiently mature into skeletal muscle cells in the transplanted tissue site to exhibit a therapeutic effect.

The skeletal muscle progenitor cells or cell group comprising skeletal muscle progenitor cells to be used for the invention may be derived from pluripotent stem cells.

Pluripotent stem cells to be used for the invention may be stem cells capable of differentiating to all cell types in the body while having proliferation potency as well, and non-restrictive examples include embryonic stem (ES) cells, germline stem (GS) cells, embryonic germline (EG) cells, induced pluripotent stem (iPS) cells, embryonic stem (ntES) cells from cloned embryos obtained by nuclear transfer, and Muse cells. Preferred pluripotent stem cells are ES cells, iPS cells and ntES cells, with human ES cells and human iPS cells being more preferred and human iPS cells being even more preferred, from the viewpoint of use in treatment of myogenic disease.

### (A) Embryonic stem cells

ES cells are stem cells having pluripotency and self-renewal proliferation potency, established from the inner cell mass of an early embryo (such as the blastocyst) of a mammal such as a human or mouse.

ES cells are embryonic stem cells derived from the inner cell mass of the blastocyst, which is the embryo after the 8-cell stage and morula stage of a fertilized egg, having the ability to differentiate into the various types of cells of the body, i.e. pluripotent differentiating power, and self-renewal proliferation potency. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165).

ES cells can be established by extracting the inner cell mass from the blastocyst of a fertilized egg of an animal and culturing the inner cell mass on a fibroblast feeder. Maintenance of the cells by subculturing can be carried out using culture medium with addition of a substance such as leukemia inhibitory factor (LIF) or basic fibroblast growth factor (bFGF). Methods for establishing and maintaining human and monkey ES cells are described in USP5,843,780; Thomson JA, et al. (1995), Proc. Natl. Acad. Sci. USA, 92:7844-7848; Thomson JA, et al. (1998), Science 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; Klimanskaya I, et al. (2006) and Nature 444:481-485, for example.

Human ES cells can be maintained using culture medium for preparation of ES cells, such as DMEM/F-12 culture medium supplemented with 0.1 mM 2-mercaptoethanol, 0.1 mM non-essential amino acids, 2 mM L-glutamic acid, 20% KSR and 4 ng/ml bFGF, in a moist atmosphere of 2% CO₂/98% air at 37°C, for example (O. Fumitaka et al. (2008), Nat. Biotechnol., 26:215-224). ES cells must be subcultured every 3 to 4 days, during which time subculturing may be carried out using 0.25% trypsin and 0.1 mg/ml collagenase IV in PBS containing 1 mM CaCl₂ and 20% KSR.

Selection of the ES cells can generally be carried out by Real-Time PCR with expression of gene markers such as alkaline phosphatase, Oct-3/4 and Nanog as indicators. Expression of gene markers such as OCT-3/4, NANOG and ECAD can also be used as an indicator for selection of the human ES cells (E. Kroon et al. (2008), Nat. Biotechnol., 26:443-452).

As human ES cell lines, WA01(H1) and WA09(H9) can be obtained from WiCell Research Institute, and KhES-1, KhES-2 and KhES-3 can be obtained from Institute for Frontier Life and Medical Sciences, Kyoto University (Kyoto, Japan).

### (B) Germline stem cells

Germline stem cells are pluripotent stem cells derived from the testes, and they serve as a source for spermatogenesis. Similar to ES cells, these cells can also be induced to differentiate to cells of various cell series, and have properties that allow creation of a chimeric mouse when they are transplanted into a mouse blastocyst, for example (M. Kanatsu-Shinohara et al. (2003) Biol. Reprod., 69:612-616; K. Shinohara et al. (2004), Cell, 119:1001-1012). They are also capable of self-renewal in culture medium containing glial cell line-derived neurotrophic factor (GDNF), and their repeated subculturing under culturing conditions similar to those of ES cells allows germline stem cells to be obtained (Takebayashi, M. et al. (2008), Jikken Igaku, Vol. 26, No. 5 (Special Edition) pp.41-46, Yodosha (Tokyo, Japan).

### (C) Embryonic germ cells

Embryonic germ cells are cells with pluripotency similar to ES cells, being established from primordial germ cells at the embryonic stage, and they can be established by culturing primordial germ cells in the presence of substances such as LIF, bFGF and stem cell factors (Y. Matsui et al. (1992), Cell, 70:841-847; J.L. Resnick et al. (1992), Nature, 359:550-551).

### (D) Induced pluripotent stem cells

Induced pluripotent stem (iPS) cells are artificial stem cells derived from somatic cells, having properties almost equivalent to those of ES cells, such as pluripotent differentiating power and self-renewal proliferation potency, and they can be created by introducing specific reprogramming factors in the form of DNA or protein into somatic cells (K. Takahashi and S. Yamanaka (2006), Cell, 126:663-676; K. Takahashi et al. (2007), Cell, 131:861-872; J. Yu et al. (2007), Science, 318:1917-1920; Nakagawa, M. et al., Nat. Biotechnol. 26:101-106(2008); International Patent Publication WO2007/069666). Reprogramming factors may consist of genes specifically expressed in ES cells, or those gene products or non-coding RNA, or genes that play important roles in maintaining non-differentiation of ES cells, or their gene products or non-coding RNA or low molecular compounds. Examples of genes among reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tell, beta-catenin, Lin28b, Sall1, Sall4, Esrrb, Nr5a2 and Tbx3, any one of which reprogramming factors may be used alone or in combinations. Examples of combinations of reprogramming factors include those described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al. (2008), Nat. Biotechnol., 26:795-797, Shi Y, et al. (2008), Cell Stem Cell, 2:525-528, Eminli S, et al. (2008), Stem Cells. 26:2467-2474, Huangfu D, et al. (2008), Nat. Biotechnol. 26:1269-1275, Shi Y, et al. (2008), Cell Stem Cell, 3, 568-574, Zhao Y, et al. (2008), Cell Stem Cell, 3:475-479, Marson A. (2008), Cell Stem Cell, 3, 132-135, Feng B, et al. (2009), Nat Cell Biol. 11:197-203, R.L. Judson et al., (2009), Nat. Biotech., 27:459-461, Lyssiotis CA, et al. (2009), Proc Natl Acad Sci USA. 106:8912-8917, Kim JB, et al. (2009), Nature 461:649-643, Ichida JK, et al. (2009), Cell Stem Cell. 5:491-503, Heng JC, et al. (2010), Cell Stem Cell. 6:167-74, Han J, et al. (2010), Nature 463:1096-100 and Mali P, et al. (2010), Stem Cells. 28:713-720.

Reprogramming factors also include factors used to increase establishment efficiency, such as histone deacetylase (HDAC) inhibitor [for example, low molecular inhibitors such as valproic acid (VPA), tricostatin A, sodium butyrate, MC1293, M344, or nucleic acid expression inhibitors including siRNA and shRNA for HDAC (such as HDAC1 siRNA Smartpool (Millipore), HuSH 29mer shRNA Constructs against HDAC1 (OriGene)], MEK inhibitors (such as PD184352, PD98059, U0126, SL327 and PD0325901), Glycogen synthase kinase-3 inhibitors (such as Bio and CHIR99021), DNA methyltransferase inhibitors (such as 5-azacytidine), histone methyltransferase inhibitors (including low molecular inhibitors such as BIX-01294, and nucleic acid expression inhibitors such as siRNA and shRNA for Suv39 hl, Suv39 h2, SetDBl and G9a), L-channel calcium agonists (such as Bayk8644), butyric acid, TGFβ inhibitors or ALK5 inhibitors (such as LY364947, SB431542,616453 and A-83-01), p53 inhibitors (such as siRNA and shRNA for p53), ARID3A inhibitors (such as siRNA and shRNA for ARID3A), miRNA such as miR-291-3p, miR-294, miR-295 and mir-302, Wnt Signaling (such as soluble Wnt3a), neuropeptide Y, prostaglandins (such as prostaglandin E2 and prostaglandin J2), and hTERT, SV40LT, UTF1, IRX6, GLIS1, PITX2 or DMRTB1, and for the purpose of the present specification, these factors used to improve establishment efficiency are not particularly distinguished from reprogramming factors.

When in the form of proteins, reprogramming factors can be introduced into somatic cells by methods such as lipofection, fusion with cell membrane-permeable peptides (such as HIV-derived TAT and polyarginine) or microinjection.

When in the form of DNA, it can be introduced into somatic cells by a method using a vector such as a virus, plasmid or artificial chromosome, or using lipofection, liposomes or microinjection. A viral vector may be a retrovirus vector, a lentivirus vector (Cell, 126, pp.663-676, 2006; Cell, 131, pp.861-872, 2007; Science, 318, pp.1917-1920, 2007), an adenovirus vector (Science, 322, 945-949, 2008), an adeno-associated virus vector or a Sendai virus vector (WO2010/008054). Examples of artificial chromosome vectors include human artificial chromosome (HAC), yeast artificial chromosome (YAC) and bacterial artificial chromosomes (BAC, PAC). Plasmids to be used include mammalian cell plasmids (Science, 322:949-953, 2008). A vector may contain a control sequence such as a promoter, enhancer, ribosome-binding sequence, terminator or polyadenylated site to allow expression of the nuclear reprogramming substance, and if necessary it may also include a selective marker sequence such as a drug resistance gene (a kanamycin resistance gene, ampicillin resistance gene or puromycin resistance gene, for example), a thymidine kinase gene or a diphtheria toxin gene, or a reporter gene sequence such as green fluorescent protein (GFP), β-glucuronidase (GUS) or FLAG. The vector may also have the LoxP sequence before or after it, in order to cleave the gene coding for the reprogramming factor or a promoter with the gene coding for the reprogramming factor bonded to it, after its transfer into somatic cells.

When in the form of RNA, it may be introduced into somatic cells by a method such as lipofection or microinjection, and in order to reduce degradation, the RNA may incorporate 5-methylcytidine and pseudouridine (TriLink Biotechnologies) (Warren L, (2010), Cell Stem Cell. 7:618-630).

The culture medium for inducing iPS cells may be DMEM, DMEM/F12 or DME culture medium containing 10 to 15% FBS (further containing, as appropriate, LIF, penicillin/streptomycin, puromycin, L-glutamine, non-essential amino acids and β-mercaptoethanol), or a commercially available culture medium [such as Mouse ES cell culture medium (TX-WES culture medium by TronboX), Primate ES cell culture medium (primate ES/iPS cells culture medium, by ReproCell), or serum-free medium (mTeSR, by Stemcell Technology)].

The culturing method may comprise contacting the somatic cells and reprogramming factor on 10% FBS-containing DMEM or DMEM/F12 culture medium in the presence of 5% CO₂ at 37°C and culturing for about 4 to 7 days, and then reseeding the cells onto feeder cells (such as mitomycin C-treated STO cells or SNL cells), culturing in bFGF-containing primate ES cell culture medium from about 10 days after contact between the somatic cells and reprogramming factor, and producing iPS-like colonies at about 30-45 days after contact, or thereafter.

Alternatively, culturing may be carried out with 10% FBS-containing DMEM culture medium (also including LIF, penicillin/streptomycin, puromycin, L-glutamine, non-essential amino acids or β-mercaptoethanol as appropriate) on feeder cells (such as mitomycin C-treated STO cells or SNL cells) in the presence of 5% CO₂ at 37°C, to produce ES-like colonies from about 25-30 days or thereafter. Examples of preferred methods are the use of, instead of feeder cells, the somatic cells themselves that are to be reprogrammed (Takahashi K, et al. (2009), PLoS One.4:e8067 or WO2010/137746), or an extracellular matrix (Laminin-5 (WO2009/123349) and Matrigel (BD Co.), for example).

A method of culturing using serum-free medium may also be used (Sun N, et al. (2009), Proc Natl Acad Sci USA, 106:15720-15725). For increased establishment efficiency, the iPS cells may be established under low-oxygen conditions (an oxygen concentration of between 0.1% and 15%) (Yoshida Y, et al. (2009), Cell Stem Cell. 5:237-241 or WO2010/013845).

Exchange with fresh culture medium may be carried out once a day from day 2 of culturing onward during the culturing period. The somatic cell count for the cells used for nuclear reprogramming is not restricted and may be in the range of about 5 × 10³ to about 5 × 10⁶ cells per 100 cm² of culture dish.

The iPS cells can be selected based on the type of colonies formed. By introducing a marker gene which is a drug resistance gene that is expressed together with a gene expressed after reprogramming of somatic cells (such as Oct3/4 or Nanog), it is possible to select the established iPS cells by culturing in solution containing the corresponding drug (selective culture medium). Selection of the iPS cells is possible by observation under a fluorescent microscope when the marker gene is a fluorescent protein gene, by addition of a luminescent substrate when it is a luciferase gene, or by addition of a chromogenic substrate when it is a color-developing enzyme gene.

The terms "somatic cells" as used herein refers to any type of animal cells (preferably mammalian cells including those of humans) other than germline cells or totipotent cells such as ova, oocytes or ES cells. Somatic cells include fetal somatic cells, neonatal somatic cells, and mature healthy or diseased somatic cells, without being limitative, and also include primary cultured cells, subcultured cells and established cell lines. Specific examples of somatic cells include (1) tissue stem cells (somatic stem cells) such as neural stem cells, hematopoietic stem cells, mesenchymal stem cells and dental pulp stem cells, (2) tissue progenitor cells and (3) differentiated cells such as lymphocytes, epithelial cells, endothelial cells, muscle cells, fibroblasts (including skin cells), hair cells, hepatocytes, gastric mucosal cells, enterocytes, splenocytes, pancreatic cells (including exocrine pancreatic cells), brain cells, lung cells, renal cells and adipocytes.

According to the invention there are no particular restrictions on the mammalian individual from which the somatic cells are to be harvested, but it is preferably a human. When the iPS cells are to be used for regenerative medicine in a human, it is particularly preferred to harvest the somatic cells from the patient or from another individual with the same or substantially the same HLA type, from the viewpoint of avoiding rejection reaction. The condition "substantially the same" for the HLA type means that the HLA matches to a degree allowing engraftment when cells obtained by inducing differentiation from somatic cell-derived iPS cells are transplanted into the patient, by use of an immunosuppressive agent or the like. For example, this includes cases of primarily HLA matching (the 3 gene loci HLA-A, HLA-B and HLA-DR, or 4 gene loci including HLA-C) (same hereunder).

### (E) ES cells derived from cloned embryo obtained by nuclear transfer

ntES cells are ES cells derived from a clone embryo prepared by nuclear transfer, and they have approximately the same properties as fertilized egg-derived ES cells (T. Wakayama et al. (2001), Science, 292:740-743; S. Wakayama et al. (2005), Biol. Reprod., 72:932-936; J. Byme et al. (2007), Nature, 450:497-502). In other words, ntES (nuclear transfer ES) cells are ES cells established from the inner cell mass of a cloned embryo-derived blastocyst obtained by exchanging an unfertilized egg nucleus with a somatic cell nucleus. Combinations of nuclear transfer (J.B. Cibelli et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus may be implanted into a mammalian enucleated unfertilized egg and cultured for several hours for reprogramming.

### (F) Multilineage-differentiating Stress Enduring cells (Muse cells)

Muse cells are pluripotent stem cells produced by the method described in WO2011/007900, and specifically they are pluripotent cells obtained by subjecting fibroblasts or marrow stromal cells to prolonged trypsin treatment, which is preferably trypsin treatment for 8 hours or 16 hours, and then suspension culturing them, resulting in SSEA-3- and CD105-positive cells.

According to one embodiment, the skeletal muscle progenitor cells or cell group comprising skeletal muscle progenitor cells to be used for the invention may be skeletal muscle progenitor cells or a cell group comprising skeletal muscle progenitor cells differentiated from iPS cells that have been induced using somatic cells harvested from the subject, or from another individual with the same or substantially the same HLA type.

Examples of myogenic diseases that may be treated by the invention include muscular dystrophy (for example, Duchenne's muscular dystrophy (DMD), Becker's dystrophy, congenital muscular dystrophy, limb girdle muscular dystrophy and myotonic muscular dystrophy), hereditary myopathy such as congenital myopathy, distal myopathy and mitochondrial myopathy, non-hereditary myopathic muscular dystrophy such as polymyositis, dermatomyositis and myasthenia gravis, glycogen storage disease, and periodic paralysis. Muscular dystrophy is a more preferred target of treatment.

According to one embodiment, the composition of the invention may be combined with a pharmaceutically acceptable carrier by conventional means to produce a parenteral dosage form such as an injection, suspending agent or drops. Examples of pharmaceutically acceptable carriers that may be included in parenteral dosage forms include aqueous solutions for injection in physiological saline, or isotonic solutions including glucose or other adjuvants (such as D-sorbitol, D-mannitol and sodium chloride). The composition of the invention may also include a buffering agent (such as phosphate buffer or sodium acetate buffer), a soothing agent (such as lidocaine hydrochloride or procaine hydrochloride), a stabilizer (such as human serum albumin or polyethylene glycol), a preservative (such as sodium benzoate or benzalkonium chloride) or an antioxidant (such as ascorbic acid or sodium edetate).

According to one embodiment, when the composition of the invention is formulated as an aqueous suspension, it may have the skeletal muscle progenitor cells suspended at about 1.0 × 10⁵ to about 1.0 × 10⁹ cells/mL in the aqueous solution. A formulation obtained in this manner will be stable with low toxicity, and can thus be safely administered to a mammal such as a human. The method of administration is not particularly restricted but is preferably injection or drip infusion, though it may also be intravenous administration, intraarterial administration or intramuscular administration (local administration to the affected site). The dose for an agent of the invention will differ depending on the individual to which it is to be administered and their symptoms, the target site for treatment and the method of administration, but for intramuscular administration (local administration to the affected site), in the case of most DMD patients (~60 kg body weight), it is convenient to administer about 1.0 × 10⁵ to 1.0 × 10⁹ cells to about 10-20 sites, as one dose of skeletal muscle progenitor cells, about 1 to 4 times at intervals of about 1 month or longer.

According to one embodiment, the invention provides a method for treating a subject suffering from myogenic disease, the method comprising:
administration of a cell group comprising skeletal muscle progenitor cells that are Pax7-positive and are positive for CD146 (MCAM) and negative for CD57 on the cell surfaces, to a subject in need thereof.

According to another embodiment, the invention provides a cell group for use in treatment of myogenic disease, the cell group comprising skeletal muscle progenitor cells that are Pax7-positive and are positive for CD146 (MCAM) and negative for CD57 on the cell surfaces.

According to another embodiment, the invention provides the use of a cell group comprising skeletal muscle progenitor cells for treatment of myogenic disease,
wherein the cell group comprises skeletal muscle progenitor cells that are Pax7-positive and are positive for CD146 (MCAM) and negative for CD57 on the cell surfaces.

### <Method for inducing differentiation of skeletal muscle progenitor cells from pluripotent stem cells>

The skeletal muscle progenitor cells or cell population comprising skeletal muscle progenitor cells to be used for the invention may be skeletal muscle progenitor cells or a cell population comprising skeletal muscle progenitor cells obtained by the method described in International Patent Publication No. WO2016/108288, for example, though this is not limitative. International Patent Publication No. WO2016/108288 is incorporated herein by reference.

For example, the method for inducing differentiation of skeletal muscle progenitor cells from pluripotent cells may be a method comprising the following step (1) and step (2A) or (2B).
(1) A step of culturing pluripotent stem cells in culture medium containing a TGF-β inhibitor and a GSK3β inhibitor;
(2A) a step of culturing the cells obtained in step (1) in culture medium containing HGF and also optionally containing IGF1;
(2B) a step of culturing the cells obtained in step (1) in the following order:
   (i) in culture medium containing IGF1, HGF and bFGF,
   (ii) in culture medium containing IGF1,
   (iii) in culture medium containing IGF1 and HGF.

The method for inducing differentiation of skeletal muscle progenitor cells from pluripotent cells may also be a method comprising a step of culturing cells obtained in the steps from step (1) and step (2A) or (2B), and further step (3) a step of culturing cells obtained in the step (2A) or (2B), in culture medium comprising a TGF-β inhibitor, IGF1 and serum.

The method for inducing differentiation of skeletal muscle progenitor cells from pluripotent cells will now be described in detail.

### Step (1): Culturing pluripotent stem cells in culture medium containing TGF-β inhibitor and GSK3β inhibitor.

Step (1) is a step of culturing pluripotent stem cells in culture medium containing a TGF-β inhibitor and a GSK3β inhibitor. The culture medium used in step (1) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, and mixtures of these media. A mixture of IMDM medium and Ham's F12 medium is preferred for step (1).

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium in step (1) is a mixture of IMDM medium and Ham's F12 medium containing added albumin, transferrin, fatty acids, insulin, selenium and thiolglycerol.

According to the invention, the TGFβ inhibitor is not particularly restricted so long as it is a substance that inhibits signal transduction from receptor-binding of TGFβ through to SMAD, and it may be a substance that inhibits binding of TGFβ to ALK family receptor, or a substance that inhibits phosphorylation of SMAD by ALK family receptor. Examples of TGFβ inhibitors for the invention include Lefty-1 (NCBI Accession Nos. NM_010094 (mouse), NM_020997 (human)), SB431542, SB202190 (both R.K. Lindemann et al., Mol. Cancer, 2003, 2:20), SB505124 (GlaxoSmithKline), NPC30345, SD093, SD908, SD208 (Scios), LY2109761, LY364947, LY580276 (Lilly Research Laboratories), A-83-01 (WO2009/146408, and derivatives of the same.

The TGFβ inhibitor to be used in step (1) is preferably SB431542.

The concentration of SB431542 in the medium is not particularly restricted but is preferably 1 µM to 50 µM, such as 1 µM, 2 µM, 3 µM, 4 µM, 5 µM, 6 µM, 7 µM, 8 µM, 9 µM, 10 µM, 11 µM, 12 µM, 13 µM, 14 µM, 15 µM, 16 µM, 17 µM, 18 µM, 19 µM, 20 µM, 25 µM, 30 µM, 35 µM, 40 µM, 45 µM or 50 µM, without being restrictive. It is more preferably 2 µM to 10 µM, and most preferably 5 µM.

The GSK-3β inhibitor of the invention is defined as a substance that inhibits GSK-3β protein kinase activity (for example, phosphorylation of β-catenins), many of which are already known including the originally discovered GSK-3β inhibitor lithium chloride (LiCl), the indirubin derivative BIO (also known as GSK-3β inhibitor IX; 6-bromoindirubin 3'-oxime), the maleimide derivative SB216763 (3-(2,4-dichlorophenyl)-4-(1-methyl-1H-indol-3-yl)-1H-pyrrole-2,5-dione), the phenyl α-bromomethyl ketone compound GSK-3β inhibitor VII (4-dibromoacetophenone), the cell membrane-permeable phosphorylated peptide L803-mts (also known as GSK-3β peptide inhibitor; Myr-N-GKEAPPAPPQSpP-NH2) and the highly selective CHIR99021 (6-[2-[4-(2,4-Dichlorophenyl)-5-(4-methyl-1H-imidazol-2-yl)pyrimidin-2-ylamino]ethylamino]pyridine-3-carbonitrile). These compounds are readily available on the market from Calbiochem or Biomol Co., for example, but they may be acquired from other sources or specially produced.

The GSK-3β inhibitor to be used in step (1) is preferably CHIR99021.

The concentration of CHIR99021 in the medium is not particularly restricted but is preferably a concentration of higher than 1 µM as a concentration used for GSK-3β inhibition, and it may be 2 µM, 3 µM, 4 µM, 5 µM, 6 µM, 7 µM, 8 µM, 9 µM, 10 µM, 15 µM, 20 µM, 25 µM, 30 µM, 35 µM, 40 µM, 45 µM or 50 µM, without being limitative. It is more preferably 5 µM or greater (such as 5 µM to 50 µM and preferably 5 µM to 10 µM).

The culturing period for step (1) may be from 10 to 30 days, for example, such as 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days or 30 days, with the preferred period being 16 days to 21 days, and especially 16 days.

### Step (2A): Culturing in culture medium containing HGF and optionally IGF1

Step (2A) is a step of culturing the cells obtained in step (1) in culture medium containing HGF and optionally IGF1. The culture medium used in step (2A) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, SF-O3 medium (Eidia) and mixtures of these media. The medium used in step (2A) is preferably SF-O3 medium.

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium for step (2) is SF-O3 medium containing added albumin and 2-mercaptoethanol.

The concentration of HGF in the medium used for step (2A) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

In step (2A) the culture is preferably carried out in culture medium containing IGF1 in addition to HGF. The concentration of IGF1 in the medium used for step (2A) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The culturing period for step (2A) may be from 1 to 40 days, for example, such as 1 day, 2 days, 3 days, 4 days, 5 days, 6 days, 7 days, 8 days, 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days or 40 days, and preferably 20 days.

### Step (2B): Successive culturing (i) in culture medium containing IGF1, HGF and bFGF, (ii) in culture medium containing IGF1 and (iii) in culture medium containing IGF1 and HGF

Step (2B) is a step of successive culturing of the cells obtained in step (1):
(i) in culture medium containing IGF1, HGF and bFGF,
(ii) in culture medium containing IGF1, and
(iii) in culture medium containing IGF1 and HGF. This step is carried out instead of the previous step (2A). Substeps (i) to (iii) will now be explained in order.

### Substep (i): Culturing in culture medium containing IGF1, HGF and bFGF

The culture medium used in substep (i) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, SF-O3 medium (Eidia) and mixtures of these media. The medium used in substep (i) is preferably SF-O3 medium.

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium for substep (i) is SF-O3 medium containing added albumin and 2-mercaptoethanol.

The concentration of IGF1 in the medium used for substep (i) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The concentration of HGF in the medium used for substep (i) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The concentration of bFGF in the medium used for substep (i) is also not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The culturing period for substep (i) may be from 1 to 10 days, for example, such as 1 day, 2 days, 3 days, 4 days, 5 days, 6 days, 7 days, 8 days, 9 days or 10 days, with 4 days being preferred.

### Substep (ii): Culturing in culture medium containing IGF1

Substep (ii) is a step of culturing the cells obtained in substep (i) in culture medium containing IGF1. The culture medium used in substep (ii) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, SF-O3 medium, and mixtures of these media. The medium used in substep (ii) is preferably SF-O3 medium.

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium for substep (ii) is SF-O3 medium containing added albumin and 2-mercaptoethanol.

The concentration of IGF1 in the medium used for substep (ii) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The culturing period for substep (ii) may be from 1 to 10 days, for example, such as 1 day, 2 days, 3 days, 4 days, 5 days, 6 days, 7 days, 8 days, 9 days or 10 days, with 3 days being preferred.

### Substep (iii): Culturing in culture medium containing IGF1 and HGF

Substep (iii) is a step of culturing the cells obtained in substep (ii) in culture medium containing IGF1 and HGF. The culture medium used in substep (iii) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, SF-O3 medium, and mixtures of these media. The medium used in substep (iii) is preferably SF-O3 medium.

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium for substep (iii) is SF-O3 medium containing added albumin and 2-mercaptoethanol.

The concentration of IGF1 in the medium used for substep (iii) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The concentration of HGF in the medium used for substep (iii) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The culturing period for substep (iii) may be from 7 to 20 days, such as 7 days, 8 days, 9 days, 10 days, 11 days, 12 days, 13 days, 14 days, 15 days, 16 days, 17 days, 18 days, 19 days or 20 days, with 14 days being preferred.

### Step (3): Culturing in culture medium containing TGF-β inhibitor, IGF1 and serum

Step (3) is a step of culturing the cells obtained in step (2A) or (2B) in culture medium containing TGF-β inhibitor, IGF1 and serum. The culture medium used in step (3) can be prepared using animal cell-culturing medium as the basal medium. Examples of basal medium include IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, StemPro34 (Invitrogen), RPMI-base medium, and mixtures of these media. The medium used in step (3) is preferably DMEM medium.

The basal medium may contain serum or it may be serum-free. If necessary, it may also contain one or more serum substitutes such as albumin, transferrin, KnockOut Serum Replacement (KSR) (FBS serum substitute for ES cell culturing) (Invitrogen), N2 supplement (Invitrogen), B27 supplement (Invitrogen), fatty acids, insulin, selenium (sodium selenite), collagen precursor, trace elements, 2-mercaptoethanol (2ME) or thiolglycerol, and may comprise one or more other substances such as lipids, amino acids, L-glutamine, GlutaMAX (Invitrogen), non-essential amino acids, vitamins, growth factors, low molecular compounds, antibiotics, antioxidants, pyruvic acid, buffering agents and inorganic salts. The preferred basal medium for step (3) is DMEM medium containing added serum, L-glutamine and 2-mercaptoethanol. The serum to be used in step (3) is horse serum, and the concentration in the basal medium is 1 to 10%, and preferably 2%.

The TGFβ inhibitor to be used in step (3) may be the same one mentioned above, and it is preferably SB431542. The concentration of SB431542 in the medium used in step (3) is not particularly restricted but is preferably 500 nM to 50 µM, such as 500 nM, 600 nM, 700 nM, 800 nM, 900 nM, 1 µM, 2 µM, 3 µM, 4 µM, 5 µM, 6 µM, 7 µM, 8 µM, 9 µM, 10 µM, 15 µM, 20 µM, 25 µM, 30 µM, 35 µM, 40 µM, 45 µM or 50 µM, without being limitative. It is more preferably 1 µM to 20 µM, and even more preferably 5 µM.

The concentration of IGF1 in the medium used for step (3) is not particularly restricted but is preferably 1 ng/ml to 100 ng/ml, such as 1 ng/ml, 2 ng/ml, 3 ng/ml, 4 ng/ml, 5 ng/ml, 6 ng/ml, 7 ng/ml, 8 ng/ml, 9 ng/ml, 10 ng/ml, 15 ng/ml, 20 ng/ml, 25 ng/ml, 30 ng/ml, 35 g/ml, 40 ng/ml, 45 ng/ml, 50 ng/ml, 60 ng/ml, 70 ng/ml, 80 ng/ml, 90 ng/ml or 100 ng/ml, without being limitative. It is more preferably 1 ng/ml to 50 ng/ml and even more preferably 10 ng/ml.

The culturing period for step (3) may be from 9 to 50 days, and is preferably from 20 to 50 days or from 30 to 50 days. For example, it may be 9 days, 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, 32 days, 33 days, 34 days, 35 days, 36 days, 37 days, 38 days, 39 days, 40 days, 41 days, 42 days, 43 days, 44 days, 45 days, 46 days, 47 days, 48 days, 49 days or 50 days.

### <Method for purifying skeletal muscle progenitor cells and method for producing cell group comprising skeletal muscle progenitor cells>

According to one embodiment, the invention provides a method for purifying skeletal muscle progenitor cells, the method comprising:
a step of recovering a cell group that is positive for CD146 (MCAM) and negative for CD57 on the cell surfaces, from a cell population comprising skeletal muscle progenitor cells.

According to another embodiment, the invention provides a method for producing a cell group comprising skeletal muscle progenitor cells, the method comprising:
a step of recovering a cell group that is positive for CD146 (MCAM) and negative for CD57 on the cell surfaces, from a cell population comprising skeletal muscle progenitor cells.

As used herein, the phrase "cell population comprising skeletal muscle progenitor cells" refers to the cell population before application of the method of the invention, and is used herein to distinguish it from a "cell group comprising skeletal muscle progenitor cells" obtained by application of the method of the invention. A "cell population comprising skeletal muscle progenitor cells", for the purpose of the invention, may be one derived from pluripotent stem cells, such as pluripotent stem cells selected from the group consisting of ES cells, ntES cells and iPS cells.

The step of recovering a cell group that is positive for CD146 (MCAM) and negative for CD57 on the cell surfaces from a cell population comprising skeletal muscle progenitor cells may be a method that allows cells that are positive for CD146 (MCAM) and negative for CD57 on the cell surfaces to be specifically selected and recovered. It can be carried out, for example, using a binding agent which specifically binds CD146 (MCAM) and a binding agent which specifically binds CD57.

As used herein, "binding agent which specifically binds" includes a substance that selectively binds at least a portion of the target protein, such as an antibody, ligand or aptamer. According to one embodiment, the binding agent to be used for the invention is a binding agent that specifically binds CD146 (MCAM) and CD57.

According to the embodiment, an "antibody" used as a binding agent for the invention may be a polyclonal antibody, monoclonal antibody or other binding fragment that binds the target protein. The term "binding fragment", as used herein, includes a monomeric Fab fragment or monomeric Fab' fragment, or a dimeric F(ab)'2 fragment or single-chain antibody molecule (scFv).

According to one embodiment, an "aptamer" used as a binding agent for the invention is a synthetic DNA or RNA molecule or synthetic analog having the ability to bind in a specific manner to the target molecule. The aptamer to be used for the invention can be obtained, for example, by selection by repetitive *in vitro* binding to various molecular target proteins (such as CD146 or CD57), using the SELEX method (see Tuerk C., Gold L., Science, 1990, 249(4968), 505-510; Ellington AD, Szostak JW., Nature, 1990, 346(6287):818-822; US Patent No. 6,867,289; US Patent No. 5,567,588; and US Patent No. 6,699,843).

According to one embodiment, a "ligand" to be used as a binding agent for the invention is a protein capable of selectively binding the target protein, where the specific binding agent to be used for the invention is not restricted and may be the ligand Galectin-1, Laminin-411 or netrin-1 for CD146 (MCAM), or the CD57 ligand CD62L or CD62P.

According to the embodiment, the step of recovering a cell group that is positive for CD146 (MCAM) and negative for CD57 on the cell surfaces may be a fluorescence-activated cell sorting (FACS) or magnetic cell sorting (MACS) method.

For the purpose of the present specification, the "fluorescence-activated cell sorting method" is a method in which cell particles are caused to flow at high speed through an extremely narrow fluid and irradiated with laser light, measuring the fluorescence emitted by the particles (assuming that the cells are already fluorescently labeled), or scattered light, and wherein the target cells can also be isolated if the apparatus is equipped with a cell sorter. If the step of recovering the cell group according to the invention is a fluorescence-activated cell sorting method, therefore, a binding agent labeled with any desired fluorescent substance may be used.

The "magnetic cell sorting (MACS)" method, for the purpose of the invention, is a method in which magnetic beads supporting a binding agent are used to separate out the target cells by magnetic trapping. If the step of recovering the cell group according to the invention is a magnetic cell sorting method, therefore, a binding agent supported on magnetic beads can be used.

The cell group comprising skeletal muscle progenitor cells obtained by carrying out the method of the invention can treat a subject suffering from myogenic disease without inducing fibrosis at the site of application in a living body. When the method of the invention is used to produce a cell population comprising skeletal muscle progenitor cells, such as a pluripotent stem cell-derived cell population comprising skeletal muscle progenitor cells, the obtained cell group comprises 30% or more of Pax7-positive skeletal muscle progenitor cells, thereby allowing treatment of a subject suffering from myogenic disease without inducing fibrosis at the site of application in a living body.

According to one embodiment, the invention provides a kit to be used in a method for purifying skeletal muscle progenitor cells or a method for producing a cell group comprising skeletal muscle progenitor cells, the kit comprising a binding agent which specifically binds CD146 (MCAM) and a binding agent which specifically binds CD57. The kit of the invention may also comprise as appropriate the necessary components required for carrying out the method, such as magnets (in the case of MACS), rinsing buffer and/or the kit instruction manual.

According to one embodiment, the invention provides a cell group comprising skeletal muscle progenitor cells obtained by the method for purifying skeletal muscle progenitor cells or the method for producing a cell group comprising skeletal muscle progenitor cells.

The present invention will now be described in greater detail by examples, with the understanding that the invention is not limited thereto.

### EXAMPLES

### 1. Experimental materials and methods

### 1-1. Culturing of pluripotent stem cells

Human iPS cells (WJ14-s01, Ff-WJs513) provided by Prof. Yamanaka of Kyoto University were cultured in StemFit AK02N medium (Ajinomoto) on a culture vessel coated with Easy iMatrix-511 silk (Nippi).

Using the WJ14-s01 cells, the NLS-Venus-T2A sequence was linked to the 5'-end of the start codon at the Pax7 gene locus by homologous recombination according to a common method to create an iPS cell line (s01-Pax7-Venus) expressing Venus in linkage with Pax7 expression.

### 1-2. Inducing differentiation from human iPS cells to skeletal muscle stem cell group

Differentiation from human iPS cells to a skeletal muscular progenitor cell group was induced in the following manner (ref: WO2016/108288).

### Step (1a) [Day 0-14]

Culturing was carried out on a MatriGel-coated culture vessel in CDMi basal medium containing 5 µM SB431542 and 10 µM CHIR99021 (mixed medium comprising IMDM (1×) Iscove's Modified Dulbecco's Medium(+)L-Glutamine(+)25 mM HEPES (Invitrogen), with addition of 1% Albumin from bovine serum (Sigma), 1% Penicillin-Streptomycin Mixed Solution (Nacalai Tesque, Inc.), 1% CD Lipid Concentrate (Invitrogen), 1% Insulin-Transferrin-Selenium (Invitrogen) and 450 µM 1-Thioglycerol (SIGMA), and F-12 (1×) Nutrient Mixture(Ham) (+)L-Glutamine (Invitrogen), in a ratio of 1:1). Subculturing was carried out on days 7 and 14 by a common method.

### Step (1b) [Day 15-16]

The cell medium obtained in step (1a) was exchanged with CDMi basal medium and culturing was continued.

### Step (2a) [Day 17-20]

The cell medium obtained in step (1b) was exchanged with SF-O3 medium containing added 0.2% BSA (Sigma), 200 µM 2-Mercaptoethanol, 10 ng/mL IGF-1(Peprotech), 10 ng/mL HGF (Peprotech) and 10 ng/mL bFGF (OrientalBio), and culturing was continued.

### Step (2b) [Day 21-23]

The cell medium obtained in step (2a) was exchanged with SF-O3 medium containing added 0.2% BSA (Sigma), 200 µM 2-Mercaptoethanol and 10 ng/mL IGF-1(Peprotech), and culturing was continued.

### Step (2c) [Day 24-37]

The cell medium obtained in step (2b) was exchanged with SF-O3 medium containing added 0.2% BSA (Sigma), 200 µM 2-Mercaptoethanol, 10 ng/mL IGF-1(Peprotech) and 10 ng/mL HGF (Peprotech), and culturing was continued.

### Step (3) [Day 38-]

The cell medium obtained in step (2c) was exchanged with medium containing added 2% Horse Serum, 1% Penicillin-Streptomycin Mixed Solution, 200 µM 2-Mercaptoethanol, 5 µM SB431542 and 10 ng/mL IGF-1, and culturing was continued until Day 80.

### 1-3. FACS analysis and sorting

Cells induced to differentiate to skeletal muscle progenitor cells by the method described above were dispersed to single cells and reacted with antibody, and analysis and sorting were carried out with a BD FACS Aria Fusion Cell Sorter (BD Bioscience). The antibodies used were APC-labeled anti-human/mouse CD207 antibody (BioLegend), BV650-labeled anti-human FGFR4 antibody (BD Biosciences), APC-labeled anti-human MCAM antibody (BioLegend), PE-labeled anti-human CD57 antibody (BD Biosciences) and Alexa Flour 647-labeled anti-human CD82 antibody (BioLegend).

Analysis and sorting of Pax7-positive cells using s01-Pax7-Venus cells was carried out using Venus as the marker.

### 1-4. RNA extraction, cDNA synthesis and quantitative PCR

The mRNA was extracted from the cells using a ReliaPrep RNA Cell Miniprep System (Promega), and cDNA was synthesized using a ReverTra Ace qPCR RT Kit (Toyobo, Ltd.). Quantitative PCR was carried out with a StepOne Plus real-time PCR system (Thermo Fisher) using cDNA, PowerSYBR Green Master Mix (Applied Biosystems) and primers.

**[Table 1]**

| Gene | Forward primer | Reverse primer |
|---|---|---|
| Pax7 | AGGGCCTCCTGCTTGTTTAT (SEQ ID NO: 1) | GGTTTTGCCCAACTCAGTGT (SEQ ID NO: 2) |
| MyoD | CACTCCGGTCCCAAATGTAG (SEQ ID NO: 3) | TTCCCTGTAGCACCACACAC (SEQ ID NO: 4) |
| TFAP2A | AGGGCCTCGGTGAGATAGTT (SEQ ID NO: 5) | AAGAGTTCACCGACCTGCTG (SEQ ID NO: 6) |
| PDGFRα | GGCCCCATTTACATCATCAC (SEQ ID NO: 7) | CATAGCTCCGTGTGCTTTCA (SEQ ID NO: 8) |
| β-actin | CTCTTCCAGCCTTCCTTCC (SEQ ID NO: 9) | CACCTTCACCGTTCCAGTTT (SEQ ID NO: 10) |

### 1-5. Inducing differentiation to myotube cells

The cells were cultured for 3 days on an iMatrix-511-coated culture vessel, in medium containing added 2% Horse Serum, 1% Penicillin-Streptomycin Mixed Solution, 200 µM 2-Mercaptoethanol, 5 µM SB431542 and 10 ng/mL IGF-1.

### 1-6. Immunostaining and measurement of myotube cells

Cells induced to differentiate to myotube cells were fixed with 2% paraformaldehyde and blocked with Blocking One (Nacalai Tesque, Inc.). The primary antibody was mouse anti-myosin heavy chain (MHC) antibody (1:800, eBioscience), the secondary antibody was Alexa568-labeled anti-mouse IgG antibody (1:500, Thermo Fisher), and nuclear staining was with 1 µg/mL DAPI (Sigma). The image was taken using a BZ-X700 All-In-One Fluorescent Microscope (Keyence), and the MHC-positive DAPI count was measured using Hybrid cell count software (Keyence), to determine the positive rate.

### 1-7. Transplanting experiment

The mice used were 6- to 8-week-old DMD/NSG mice obtained by cross-breeding severe immunodeficient mice NSG (NOD/SCID/IL2Rynull) and DMDnull mice lacking dystrophin expression. The hind limb gastrocnemius muscle of each mouse was injected with 8 × 10⁵ CD82-positive, CD57-negative cells or MCAM-positive, CD57-negative cells. The gastrocnemius muscle was sampled on day 28 after transplantation, and a frozen section was prepared.

### 1-8. Tissue section staining

The gastrocnemius muscle frozen section was fixed with 4% paraformaldehyde and blocked with Blocking One (Nacalai Tesque, Inc.). The primary antibodies used were anti-dystrophin antibody (1:500, Abcam), anti-lamin A/C antibody (1:200, Leica) and anti-laminin α2 antibody (1:50, Alexis), the secondary antibodies used were Alex488-labeled anti-mouse IgG2b antibody, Alex568-labeled anti-mouse IgG1 antibody and Alex647-labeled anti-rat IgG antibody, and nuclear staining was with 1 µg/mL DAPI (Sigma).

HE staining was carried out using hematoxylin (Merck KGaA) and eosin (Merck KGaA).

Sirius Red staining was carried out using a Picrosirius Red Stain Kit (Polysciences). The image was taken using a BZ-X700 All-In-One Fluorescent Microscope (Keyence), and the collagen stained area was measured using Hybrid cell count software (Keyence), to determine the fibrosis area percentage.

### 1-9. RNA sequencing

After extracting the mRNA from the cells with a ReliaPrep RNA Cell Miniprep System (Promega), a cDNA library prepared using a TruSeq Strand mRNA Library Prep Kit (Illumina) was sequenced with a NextSeq 500/550 v2.5 kit. The sequencing results were converted to FASTQ format using BCL2FASTQ Conversion Software 1.8.4, and then rpmkforgenes script was used for quantitation. The quantitation results were analyzed using Genespring GX 13 software.

### 2. Results

### 2-1. Search for skeletal muscle stem cell markers

Pax7-positive, CD57-negative cells and Pax7-negative cells were sorted from a skeletal muscular progenitor cell group obtained by inducing differentiation of human iPS cells (s01-Pax7-Venus) expressing the fluorescent protein Venus in linkage with Pax7 expression, and the gene expression level was measured using an RNA sequencer. CD207, FGFR4 and MCAM were found as candidate markers with high gene expression in the Pax7-positive, CD57-negative cells, and with proteins localized in the cell membranes (Fig. 1).

The positive rate for CD207, FGFR4 and MCAM in the Pax7-positive cells was then examined. As a result, 98% of the Pax7-positive cells were found to be MCAM-positive (Fig. 2).

The purity and yield were then examined for sorting Pax7-positive cells by MCAM from among the skeletal muscular progenitor cell group obtained by inducing differentiation of iPS cells. The purity and yield of Pax7-positive cells among the MCAM-positive, CD57-negative cells were 56% and 76%, respectively. This was a major improvement over the previously reported cell surface marker CD82 for sorting skeletal muscle progenitor cells (Matthew S. Alexander, et al., Cell Stem Cell 19, 1, 2016) (Table 2).

**[Table 2]**

| Fraction | Purity (%) | Yield (%) |
|---|---|---|
| CD57-negative | 32 | 91 |
| CD82-positive | 36 | 81 |
| MCAM-positive | 32 | 94 |
| CD82-positive, CD57-negative | 43 | 66 |
| MCAM-positive, CD57-negative | 56 | 76 |

Table 2: Results of FACS analysis of skeletal muscular progenitor cell group induced to differentiate from human iPS cells expressing fluorescent protein Venus in linkage with Pax7 expression. The purity was calculated from the number of Pax7-positive cells among the total number of cells in each fraction. The yield was calculated from the number of Pax7-positive cells in each fraction among the total number of Pax7-positive cells.

### 2-2. Phenotype of MCAM-positive, CD57-negative cells in vitro

The gene expression levels of the Pax7, MyoD, TFAP2A and PDGFRα genes were quantified after sorting out CD57-positive cells, MCAM-positive, CD57-negative cells and MCAM-negative, CD57-negative cells from a skeletal muscular progenitor cell group obtained by inducing differentiation of human iPS cells. The skeletal muscular progenitor cell marker Pax7 and the skeletal muscle cell marker MyoD were highly expressed in the MCAM-positive, CD57-negative cells. In contrast, the non-skeletal muscle cell markers TFAP2A (neural crest cell marker) and PDGFRα (mesenchymal stem cell marker) had low expression in the MCAM-positive, CD57-negative cells (Fig. 3).

After then sorting out CD57-positive cells, MCAM-positive, CD57-negative cells and MCAM-negative, CD57-negative cells from among a skeletal muscular progenitor cell group obtained by inducing differentiation of human iPS cells, the differentiation potency to myotube cells was examined. The MCAM-positive, CD57-negative cells differentiated to myotube cells stained with MHC, with an MHC positive rate of about 80% (Fig. 4).

### 2-3. MCAM-positive, CD57-negative cell transplanting test

The effect of transplanting CD82-positive, CD57-negative cells and MCAM-positive, CD57-negative cells into DMD/NSG mice was examined. At 4 weeks after transplantation, the tissue sections were immunostained for dystrophin and the engraftment and muscle regenerative power of the transplanted cells were evaluated. Both the CD82-positive, CD57-negative cells and the MCAM-positive, CD57-negative cells were found to fuse with the host muscular fibers, producing about the same number of dystrophin-positive muscular fibers (Fig. 5).

With HE staining of the tissue sections at the transplanted sites, interstitial expansion and disturbed alignment of muscular fibers were observed in the CD82-positive, CD57-negative cell-transplanted tissue, whereas muscular fiber histology was normal in the MCAM-positive, CD57-negative cell-transplanted tissue (Fig. 6).

With Sirius Red staining of the tissue sections at the transplanted sites, fibrosis was accelerated in the interstitial sections that had expanded in the tissue with CD82-positive, CD57-negative cell transplantation, whereas no abnormal fibrosis was seen in the tissue where MCAM-positive, CD57-negative cells had been transplanted (Fig. 7).

[Sequence Listing]

## Claims

1. Skeletal muscle progenitor cells that are positive for Pax7, and that are positive for CD146 (MCAM) and negative for CD57 on the cell surfaces.

2. The skeletal muscle progenitor cells according to claim 1, which are derived from pluripotent stem cells.

3. The skeletal muscle progenitor cells according to claim 2, wherein the pluripotent stem cells are selected from the group consisting of ES cells, ntES cells and iPS cells.

4. The skeletal muscle progenitor cells according to any one of claims 1 to 3, wherein the skeletal muscle progenitor cells are capable of differentiating into myosin heavy chain (MHC)-positive skeletal muscle cells and further maturing into multinucleated skeletal muscle cells in a culture system.

5. A composition for treating myogenic disease, which includes a cell group comprising skeletal muscle progenitor cells according to any one of claims 1 to 4.

6. The composition according to claim 5, wherein the cell group comprises 30% or more of the skeletal muscle progenitor cells.

7. The composition according to claim 5 or 6, which does not induce fibrosis at the site of application in a living body.

8. The composition according to any one of claims 5 to 7, wherein the myogenic disease is muscular dystrophy.

9. The composition according to any one of claims 5 to 8, which includes a pharmaceutically acceptable carrier.

10. A method for purifying skeletal muscle progenitor cells, the method comprising:
a step of recovering a cell group that is positive for CD146 (MCAM) and negative for CD57 on the cell surfaces, from a cell population comprising skeletal muscle progenitor cells.

11. The method according to claim 10, wherein the cell group comprises 30% or more of the Pax7-positive skeletal muscle progenitor cells.

12. The method according to claim 10 or 11, wherein the step is performed by a fluorescence-activated cell sorting (FACS) or magnetic cell sorting (MACS) method.

13. The method according to any one of claims 10 to 12, wherein the cell population is derived from pluripotent stem cells.

14. The method according to claim 13, wherein the pluripotent stem cells are selected from the group consisting of ES cells, ntES cells and iPS cells.

15. A kit for use in the method according to any one of claims 10 to 14, the kit comprising a binding agent which specifically binds CD146 (MCAM) and a binding agent which specifically binds CD57.

16. A method for producing a cell group comprising skeletal muscle progenitor cells, the method comprising:
a step of recovering a cell group that is positive for CD146 (MCAM) and negative for CD57 on the cell surfaces, from a cell population comprising skeletal muscle progenitor cells.

17. The method according to claim 16, wherein the cell group comprises 30% or more of the Pax7-positive skeletal muscle progenitor cells.

18. The method according to claim 16 or 17, wherein the step is performed by a fluorescence-activated cell sorting (FACS) or magnetic cell sorting (MACS) method.

19. The method according to any one of claims 16 to 18, wherein the cell population is derived from pluripotent stem cells.

20. The method according to claim 19, wherein the pluripotent stem cells are selected from the group consisting of ES cells, ntES cells and iPS cells.

21. A kit for use in the method according to any one of claims 16 to 20, the kit comprising a binding agent which specifically binds CD146 (MCAM) and a binding agent which specifically binds CD57.

22. A cell group comprising skeletal muscle progenitor cells, obtained by the method according to any one of claims 16 to 20.
